(19) Europäisches Patentamt
European Patent Office
Office européen des brevets

(11) Publication number: 0 221 505 B1

# (12) EUROPEAN PATENT SPECIFICATION

(45) Date of publication of patent specification: 14.08.91

(51) Int. Cl.$^5$: A61K 39/395, C12N 11/06, C12N 11/08, C12N 11/10, C12N 11/14

(21) Application number: 86115016.7

(22) Date of filing: 29.10.86

## (54) Method for the preparation of immunoglobulins suitable for intravenous administration.

(30) Priority: 08.11.85 IT 2276685

(43) Date of publication of application: 13.05.87 Bulletin 87/20

(45) Publication of the grant of the patent: 14.08.91 Bulletin 91/33

(84) Designated Contracting States: AT BE CH DE ES FR GB GR IT LI LU NL SE

(56) References cited:

EP-A- 0 110 995

CHEMICAL ABSTRACTS, vol. 94, no. 20, 18th May 1981, page 389, abstract no. 162780x, Columbus, Ohio, US; & JP-A-81 15 215 (JAPANESE RED CROSS SOCIETY) 14-02-1981

APPLIED BIOCHEMISTRY AND BIOTECHNOLOGY, vol. 11, no. 3, 1985, pages 191-193; M. WILCHEK et al.: "Activation of sepharose with N,N'-disuccinimidyl carbonate"

CHEMICAL ABSTRACTS, vol. 101, no. 13, 24th September 1984, page 287, abstract no. 106515r, Columbus, Ohio, US; K.M. SHAMSUZZAMAN et al.: "Immobilization of pepsinogen on Sepharose", & BIOTECHNOL. LETT. 1984, 6(6), 351-6

(73) Proprietor: SCLAVO S.p.A.
Via Fiorentina 1
I-53100 Siena(IT)

(72) Inventor: Gazzei, Guido
Via Dante, 36
Siena(IT)
Inventor: Giannozzi, Aldo
Via Celso Cittadini, 3
Siena(IT)
Inventor: Valeri, Andrea
Via Quinto Settano, 10
Siena(IT)

(74) Representative: Gervasi, Gemma, Dr. et al
Studio Brevetti e Marchi NOTARBARTOLO & GERVASI 33, Viale Bianca Maria
I-20122 Milano(IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention refers to a method for the preparation of lyophilized immunoglobulins suitable for intravenous administration.

More particularly the present invention refers to a method for the preparation of immunoglobulins suitable for intravenous administration by treatment with pepsin immobilized on a hydrophilic gel functionalised with amino groups wherein a dicarboxylic acid disuccinimidyl ester is employed as the coupling agent.

Separation of human plasma into its different components has been studied and set up by E.J.Cohen in the U.S.A. in the early forties (E.J. Cohn et al., J.Am.Chem.Soc. 62 (1940), pp.3386-93).

Cohn Fraction II (IgG concentrate) has been used since the first half of the fifties for the immunotherapy (passive immunoprophylaxis) of patients suffering from congenital or acquired agammaglobulinemia (D.Gitlin, C.A.Janeway, Prog. Hemat. I (1956) 318-329).

The intramuscular administration of these preparations is generally well tolerated and discomfort after large-volume injections is the only common reaction reported. On the contrary, tachycardia, respiratory distress, a sensation of pressure in chest, pain and even more severe anaphylactic shock-like reactions have been produced by their intravenous administration (C.A.Janeway, The development of clinical uses of immunoglobulins. A Review in immunoglobulins, E.Merler Editor - Washington - National Academy of Sciences (1970)).

Anaphylaxis seems to be produced by the presence of IgG aggregates which form during the Cohn ethanol fractionation method. It has been confirmed in fact that immunoglobulin aggregates do activate the complement system aspecifically (C.A.Janeway, ibidem).

Since IgG intravenous infusion would provide a highly desirable therapy, especially when an immediate rise in antibodies is required, several methods have been developed to provide immunoglobulin preparations suitable for intravenous administration. The most interesting ones are those listed below :

- IgG proteolysis by pepsin
  (H.E. Schuitze; G. Schwick - Veber Neve Moglichkeiten Intravenoser Gammaglobulin Applikation Desch. Med. Wschr. 87, 1643 (1962))
- IgG proteolysis by plasmin
  (J.T.Sgouris; Vox Sang. 13, 71-84, (1967))
- Chemical modification of the IgG molecule with $\beta$-propiolactone
  (W.Stephan; Vox Sang. 28, 422-437, (1975))
- Chemical modification of the IgG molecule by reduction and S-sulphonation of the disulfide bridges
  (Y. Masuho; S. Tomibe; K. Matsuzawa; A. Ohtsu; Vox Sang. 32, 175-181, (1977))
- Chemical modification of the IgG molecule by reduction-amidation
  (US Patent 4,118,379)
- Chemical modification of the IgG molecule by reduction of the disulfide bridges under controlled conditions using dithiothreitol and alkylation with iodoacetamide
  (P.M. Fernandes; J.L. Lundbland; Vox Sang, 39, 101-112, (1980))
- Removal of aggregates by precipitation with polyethylene glycols
  (A.Polson; C. Ruiz-Bravo; Vox Sang. 23, 107-118, (1972))
- IgG preparation by plasma fractionation in mild conditions (using ion exchange resins)
  (A.J.Johnson, V.E.Mac Donald et al.; J.Lab.Clin.Med. 92, 194-210, (1978) - A.D.Friesen, J.M.Bowma, W.C.M.Bees; Vox Sang. 48, 201-212 (1985))
- Treatment of IgG with trace amounts of pepsin at pH 4
  (S.Barandun, P.Kistler; Intravenous Administration of Human -Globulins; Vox Sang. 7, 157-174, (1962)-)
- Treatment of IgG with a buffer having pH 4.25
  (B.Pirofsky, The Am.J.of Med. 76 (3A), 53-60, (1984))

All these methods, however, have some disadvantages.

Those methods which are based on proteolysis by pepsin or plasmin lead to products with a molecular weight lower than that of the starting protein (and therefore with a reduced half-life and a limited biological function).

Those methods which are based on treatment with $\beta$-propiolactone or reduction followed by alkylation (or S-sulphonation) of the inter-chained -S-S- bridges, afford, as the final product, an immunoglobulin molecule which is chemically altered with respect to the native molecule. Treatment with trace amounts of soluble pepsin at pH 4 yields a product with the same molecular weight of the native molecule but contaminated with traces of an enzyme of animal origin.

A fairly high anticomplement activity has been detected in preparations containing immunoglobulins obtained by using polyethylene glycol (J.Romer et al.; Vox Sang. 42, 74-80, (1982)).

Plasma fractionation methods in mild conditions afford intact immunoglobulins substantially devoid of anticomplement activity only when the starting material is whole plasma. It is not possible, by these methods, to use as a starting material a frozen or lyophilized Cohn Fraction II with high ACA.

Furthermore precipitation with 25% ethanol is an effective method to control viral contaminations.

Treatment with a buffer pH 4.25 affords a product with a pH too far from the physiological one.

Finally, Japanese patent application no. 88715/79 in the name of The Japan Red Cross Society (Derwent - Farmdoc 38910 X) teaches a method for preparing immunoglobulins by the use of pepsin immobilized on a hydrophilic gel using glutaraldehyde as the coupling agent.

This last method however, gives a product which is proteolysed by 30%. IgG molecules, after proteolysis, are still capable of binding the antigen molecules, but are no longer capable of activating the complement system, which is responsible inter alia for bacteriolysis.

Furthermore, half-life of proteolyzed IgG is 3 days compared with 28 days of the native molecule. The disadvantages of a high percentage of proteolysed molecules are therefore apparent.

It has now surprisingly been found that it is possible to prepare lyophilized immunoglobulins suitable for intravenous administration by a method which overcomes all the above mentioned disadvantages.

Object of the present invention is therefore a method for the preparation of immunoglobulins suitable for intravenous administration by incubation with pepsin immobilized on a hydrophilic gel functionalised with amino groups, wherein a dicarboxylic acid disuccinimidyl ester of the general formula (I)

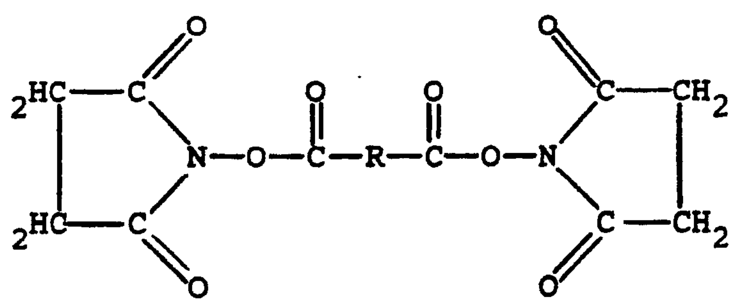

(I)

wherein R is a straight or branched alkylene radical containing from 1 to 20 carbon atoms, a

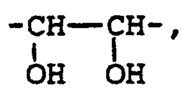 , or a 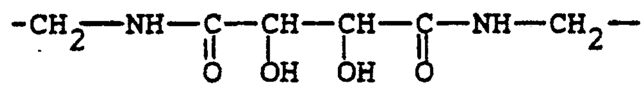

group,
is used as the coupling agent.

According to a preferred embodiment of the present invention, a dicarboxylic acid disuccinimidyl ester of formula (I) wherein R is an alkylene radical containing from 1 to 10 carbon atoms is used as the coupling agent. In a most preferred embodiment of the present invention a dicarboxylic acid disuccinimidyl ester of formula (I) wherein R is a straight alkylene radical containing from 2 to 6 carbon atoms is used as the coupling agent.

Adipic acid disuccinimidyl ester (R = $-(CH_2)_4-$) particularly useful for the scope of the present invention.

The method of the present invention affords immunoglobulins which upon lyophilization give immunoglobulin preparations particularly suitable for intravenous administration since they are :

- substantially devoid of anticomplement activity (ACA)
- not chemically altered
- not proteolysed, i.e. they have the same molecular weight of the native immunoglobulins and the same half-life
- free from any enzyme of animal origin since the immobilized pepsin is removed at the end of the process and the binding force between the enzyme and the carrier is so strong that leakage of the enzyme does not occur even in the presence of substrates or salt solutions of high ionic strenght.

In particular, the method of the present invention for preparing lyophilized immunoglobulins runs through the following steps :

a) immobilizing pepsin on a hydrophilic gel suitably functionalised with amino groups, using a dicarboxylic acid disuccinimidyl ester of formula (I) as the coupling agent;

b) incubating the obtained insolubilized pepsin with immunoglobulins;

c) recovering the immunoglobulins and lyophilizing them. As carrier for pepsin immobilization, any hydrophilic gel, either organic or inorganic, can suitably be employed provided it contains amino groups or can be functionalised by the introduction of amino groups, it cannot be proteolyzed by the enzyme itself, and is stable and inert under the reaction conditions suitably selected for the incubation step.

Generally, however, as far as the organic supports are concerned, polysaccharide derivatives, such as cellulose, dextran, agarose and polyacrylamide gel as well as their aminated derivatives and synthetic polymer gel are most suitably employed. Some are available commercially under trade names such as Sepharose, Sephadex, Bio-Gel, Enzacryl, and so forth. As for the inorganic supports, porous silica gel proved to be suitable for the present process.

Particularly preferred, also for cost and availability reasons, are the commercially available gel known as Sepharose 4B®, Cl-Sepharose 4B®, AH-Sepharose 4B®.

When the starting hydrophilic gel does not contain amino groups, these can be easily introduced therein by any of the several methods widely known in literature (see, for instance, J.Porath, R.Axen, S.Ernback, (1967), Nature, 215, 1491, or G.S.Bethell, J.S.Ayers, W.S.Mancock, M.T.W.Hearn, The Journal of Biological Chemistry, Vol.254, n.8, pp.2577-2574, 1979). However, the method reported in the latter literature reference, which uses carbonylimidazole as the condensing agent, proved to be particularly useful.

Immobilization of pepsin on the aminated hydrophilic gel using a dicarboxylic acid disuccinimidyl ester of formula (I) as the coupling agent, is carried out in two separate steps.

More particularly, the carrier bearing the amino groups is first activated through reaction with the suitably selected dicarboxylic acid disuccinimidyl ester of formula (I), and then the thus activated support is treated with pepsin.

The use of a compound of formula (I) as the coupling agent allows the reaction with the enzyme to be carried out in particularly mild conditions. This is very important as the reaction conditions required for enzyme immobilization by covalent binding are generally rather complicated and not particularly mild. Therefore, in some cases, covalent binding might alter the conformational structure and active center of the enzyme, resulting in major loss of activity and/or changes in substrate specificity.

The dicarboxylic acid disuccinimidyl esters of formula (I) are either known products or can be prepared by conventional condensation methods readily apparent to any chemist.

In the first step of the binding process, the aminated hydrophilic gel is reacted with a compound of formula (I) employed in a strong molar excess with respect to the amino groups of the support. Said molar excess is typically comprised between 5/1 and 15/1, and preferably between 8/1 and 12/1.

This reaction, which may conveniently be carried out at room temperature, is performed in the presence of an inert polar organic solvent or solvent mixture compatible with the selected support. Organic solvents which may suitably be employed in this step are for instance dioxane, tetrahydrofuran, higher aliphatic ethers, dimethylformamide and the like solvents, and , preferably, dioxane.

When the reaction, which is generally complete in a few hours, is over, the activated support is recovered and carefully washed with a buffer having a pH in the range from 5.0 to 6.2 which is suitable for the next step.

Treatment of the activated support with pepsin is in fact carried out in a buffer with a pH value in the range 5.0-6.2 and preferably 6.0-6.2. Phosphate buffers, and preferably 0.1M phosphate buffer, can suitably be employed to this purpose. The reaction may be carried out at a temperature generally comprised between 0 °C and room temperature. Preferably however the temperature is kept below 10 °C to avoid formation of bacterial contaminants.

From about 30 to about 70 mg of pepsin per ml of swollen gel can conveniently be employed to provide for an adequate cross-link binding of pepsin to the activated support.

After few hours (generally comprised between 3 and 7 and typically 4-5), when the reaction is complete, the immobilized pepsin is separated simply by filtration or decantation, and washed carefully in order to remove even traces of contaminants.

If desired, such immobilized pepsin may be stored. In this case it is preserved in acidic buffers (pH 4-4.5) optionally with the addition of small amounts of preservative agents (e.g. formaldehyde solution).

Otherwise, the immobilized enzyme can be used directly in the next incubation step.

A further object of the present invention is therefore a method for immobilizing pepsin to a hydrophilic gel functionalized with amino groups using a dicarboxylic acid disuccinimidyl ester of the general formula

(I), wherein R is as defined above, as the coupling agent.

The starting material used for the preparation of the lyophilized immunoglobulins for intravenous administration according to the present invention is frozen or lyophilized Cohn Fraction II, which is obtained from blood plasma pooled from normal or immunized donors, by successive precipitations with cold ethanol .

More particularly, before incubation with pepsin, a thorough purification is carried out by dissolving the immunoglobulins in distilled water for injection and suitably adjusting the pH and ionic strenght of the obtained solution (e.g. to a pH comprised between 6.0 and 7.0 and ionic strenght from about 0.005 to about 0.05) in order to precipitate any impurities. The insoluble can then be removed by filtration with a filter aid and the like separation procedures, and the pH of the clarified solution is then adjusted to the optimum range for the incubation step, which is comprised between 3.8 and 4.3, and preferably is about 4.

The obtained solution is then added to the immobilized enzyme. Altough batch methods are conveniently employed for the incubation of immunoglobulins with immobilized pepsin, it is also possible to circulate an immunoglobulin solution through a column containing the immobilized pepsin.

The amount of immobilized pepsin to be used in this step depends on the amount of immunoglobulins in the clarified solution. Optimum results have been obtained using an amount of immobilized pepsin which provides for an enzymatic activity of from 600 to 3.000 U/g of immunoglobulins.

Incubation is carried out at a temperature which is comprised between 34 and 40 °C, and preferably between 36 and 38°C, and takes from about 16 to 24 hours, generally from 17 to 20 hours, to be complete.

In order to warrant the best results, protein concentration is kept below 5 % and preferably below 3% (w/v).

However, in order to meet the requirements of the current industrial pharmacy practice and avoid the use of extremely large volumes, protein concentration is preferably kept above 1 % and preferably above 2% (w/v).

At the end of the incubation, the immobilized enzyme is recovered, typically by centrifugation or filtration through a porous membrane, and protein concentration and pH of the solution containing the immunoglobulins are adjusted as needed for the next lyophilization step. Lyophilization is then carried out according to <u>per se</u> known methods conventionally employed in the pharmaceutical practice for the preparation of sterile freeze-dried products.

The lyophilized immunoglobulin preparation thus obtained, which is suitable for intravenous administration has the following characteristics :

- molecular weight distribution (assayed by HPLC, according to the method described by H. Sumela et al. in J.Chromat. 297, (1984), pp. 369-373) comparable to the parent product with %monomer > 85, %dimer ≦ 12, %polymer < 5, fragmentation products ≦ 5%;
- ACA (assayed by following substantially the hemolysis method suggested by The Swiss Red Cross and described by M.M. Maier in "Kabat and Mayer's Experimental Immunochemistry", Second Edition (1961), Charles C. Thomas - Springfield - pp.133-240) always lower than 20 $CH_{50}$/g and generally lower than 15 $CH_{50}$/g;
- no chemical alteration;
- absence of contaminants;

furthermore, the characteristics of the preparation do not alter after 1 month at 37 °C (stability test).

The following examples will further illustrate the invention, altough the invention is not limited to these specific examples.

Example 1

<u>PEPSIN IMMOBILIZED ON AH-SEPHAROSE 4B®</u>

1) <u>Preparation of adipic acid disuccinimidyl ester</u>

A solution of N,N-dicyclohexylcarbodiimide (88 mmol) in dioxane (80 ml) is dripped into a 1000-ml, three-necked flask equipped with a dropping funnel and stirring means, containing a solution of adipic acid (5.84 g, 40 mmol) and N-hydroxysuccinimide (9.2 g, 80 mmol) in dioxane (320 ml). The reaction mixture is then stirred at room temperature for 18-24 hours.

Then, the obtained suspension is filtered through a porous porcelain Buchner filter (G3), the obtained solid is extracted four times with pure dioxane (50 ml + 3×100 ml). The extracting solvent is then combined with the filtrate and dioxane is removed by means of a rotary evaporator; the obtained raw material is purified by crystallization from isopropyl alcohol. The thus obtained adipic acid disuccinimidyl ester is heated to about 70°C under vacuum (water pump) to remove even traces of isopropyl alcohol. The

obtained product has been characterized by its melting point, other chemico-physical characterizing data are herein reported for information purposes. The compound is preserved in anhydrous conditions at -20° C.

| M.p. | Elemental analysis | | | ν $cm^{-1}$ | yield |
|---|---|---|---|---|---|
| | C | H | N | | |
| 168 °C | 49.41% | 8.23% | 4.70% | 1735-1785-810 | 80% |

2) Preparation of pepsin immobilized on AH-Sepharose 4B®

AH-Sepharose 4B®, (30 g) containing from about $6 \cdot 10^{-6}$ to about $10^{-5}$ moles of amino groups per ml of gel, is swollen and washed on a porous porcelain büchner with 0.5N NaCl (5000 ml) and then with water (2000 ml).

The gel is then sequentially washed with three water-dioxane mixtures (1 l each) with the following proportions 80:20, 50:50, 20:80 and finally with pure dioxane (2500 ml).

The thus washed gel is charged into a flask, equipped with a mechanical stirrer, containing adipic acid disuccinimidyl ester (6.6 g) in dioxane (200-300 ml). The diester is contained in a ratio which is about ten times higher than that of the support amino groups.

The activation reaction is allowed to proceed at room temperature for 4 hours under stirring.

As the activation reaction is over, the reaction mixture is filtered and the activated gel is recovered and washed with dioxane (500 ml) and phosphate buffer pH 6.2 (300 ml).

In all the procedures which involve the use of organic solvents it is necessary to avoid any harsh treatment of the gel, as it acquires a noteworthy attitude to conglomerate and this would be detrimental to the preparation.

The activated support is charged into a flask, equipped with a mechanical stirrer, containing a solution of pepsin (6 g) in phosphate buffer pH 6.2 (500-700 ml); the reaction mixture is stirred at 4° C for 4 hours.

When the reaction is over, the reaction mixture is filtered, the support-bound enzyme is washed with phosphate buffer pH 6.2 (300 ml), charged into a column and washed first with 6N urea adjusted to pH 4 by the addition of HCl (1500 ml) and then with 0.5N NaCl in acetate buffer pH 4 (6000 ml).

After these washings, the gel can be allowed to settle in a small graduated column.

The thus prepared gel is preserved in 0.5M NaCl in acetate buffer pH 4, containing 1% formaldehyde solution.

Example 2

Introduction of amino groups on Cl-Sepharose-4B® by means of carbonyl-diimidazole

Cl-Sepharose 4B®(10 g) is washed on a büchner with water (250 ml), three water-dioxane mixtures having the following volume ratios 80:20, 50:50, 20:80, and finally with pure dioxane (200 ml). The gel is then suspended in dioxane (30 ml) containing carbonyl-diimidazole (0.400 g); the activation reaction is over in 30 minutes at room temperature under stirring.

The activated gel is then recovered, washed with dioxane (100 ml) and water (100 ml), then it is suspended in alkalinized water pH 10 and reacted with 1,6-hexanediamine (2.9 g) for 24 hours at 4° C under stirring.

The obtained functionalised support is finally washed with a slightly acidic aqueous solution (300 ml) and then with 0.5N NaCl and preserved in this last solution at 4° C.

Example 3

Preparation of pepsin immobilized on Cl-Sepharose-4B® functionalised with amino groups

The method for immobilizing pepsin on Cl-Sepharose-4B® functionalised with amino groups is analogous to that described for the immobilization of pepsin on AH-Sepharose-4B®.

Example 4

Lyophilized immunoglobulins for intravenous administration Preparation of from 100 to 400 vials by the use

of pepsin immobilized on AH-Sepharose-4B®

1) Dissolve the immunoglobulins (either lyophilized or frozen paste Cohn Fraction II) in distilled water for injections up to a protein concentration of about 50 g/kg
2) Adjust the pH to 6.5 ± 0.05 by the addition of 0.2M HCl and the ionic strenght to 0.01 by the addition of NaCl.
3) Clarify the solution by filtration adding Celite 577 (10 g/Kg) or the like filter aid, at 4° C with Millipore CWSC filters.
4) Adjust protein concentration to 22 g/Kg by the addition of distilled water for injection.
5) Adjust the pH to 4.00 ± 0.05 by the addition of 0.2M HCl.
6) Sterilize the solution by filtration in sterile flasks equipped with stirring means, with membranes such as Durapore Millipore and the like.
7) Add pepsin immobilized on AH-Sepharose-4B® obtained in example 1.
The enzyme activity which depends on the anticomplement activity of the starting material and on its resistance to proteolysis, may range from 1000 to 3000 U/g of protein.
8) Incubate for 18 hours at 37° C under mild stirring.
9) Separate the enzyme by means of a porous porcelain filter (G1-G2) and recover it.
10) Cool the solution to 20° C and then adjust the pH to 6.5 ± 0.05 by the addition of 0.2M NaOH.
11) Clarify as under 3) above.
12) Concentrate the proteins by ultrafiltration with the Pellicon system assembled with PTHK cassettes (100.000 nmwl) in view of the amount of protein needed for each vial.
13) Clarify as under 3) above.
14) Add saccarose up to an approximately isotonic solution.
15) Sterilize by filtration as under 6) above.
16) Distribute the solution in 100-ml vials (50 ml of a 6% solution, if vials containing 3 g of gammaglobulins each are desired).
17) Freeze-dry at -21° C ending at 30° C.
The obtained immunoglobulin has the following characteristics :

a molecular weight distribution similar to that of the native molecule with % monomer > 85, % dimer < 12, fragmentation products < 1%, and no detectable % of polymer (aggregate);
it is not altered chemically;
it has an anticomplement activity of 8.64 $CH_{50}$/g
it does not contain contaminants;
after one month at 37° C (stability test), it maintains the above characteristics.

Example 5
Lyophilized immunoglobulins for intravenous administration

Method for the preparation of from 100 to 400 vials by the use of pepsin immobilized on Cl-Sepharose-4B® functionalised with amino groups

The method for the preparation of lyophilized immunoglobulins for intravenous administration by the use of pepsin immobilized on C-Sepharose-4B® functionalized with amino groups is analogous to the method described above which involves the use of AH-Sepharose-4B®.

## Claims

1. A method for the preparation of immunoglobulins for intravenous administration by treatment thereof with pepsin immobilized on a hydrophilic gel functionalised with amino groups characterized in that a dicarboxylic acid disuccinimidyl ester of general formula (I)

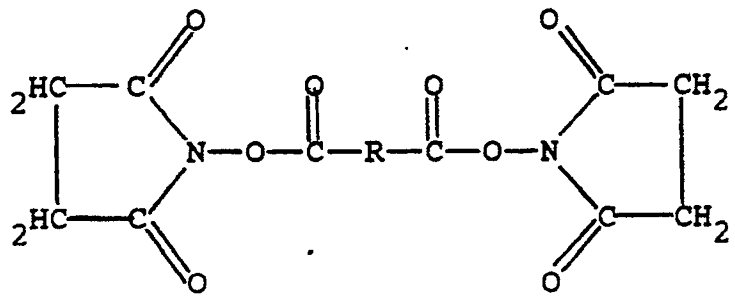

(I)

wherein R is a straight or branched alkylene radical containing from 1 to 20 carbon atoms, a

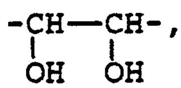 or a 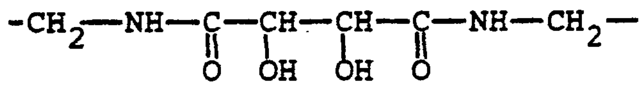

group,
is used as the coupling agent.

2. A method for preparing pepsin immobilized on a hydrophilic gel containing amino groups characterized in that a dicarboxylic acid disuccinimidyl ester of general formula (I)

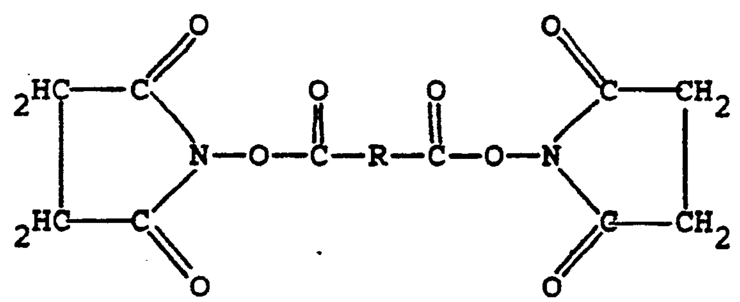

(I)

wherein R is a straight or branched alkylene radical containing from 1 to 20 carbon atoms, a

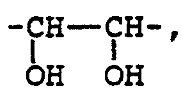 or a 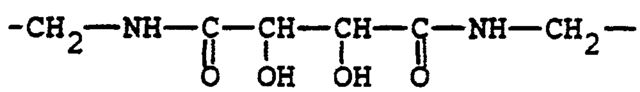

group,
is used as the coupling agent.

3. The method of claims 1 or 2 wherein the hydrophilic gel is selected from agarose gel, dextrane gel, cellulose gel, polyacrylamide gel, porous silica gel, their aminated derivatives and synthetic polymer gel.

4. The method of claim 3 wherein the hydrophilic gel is selected from Sepharose-4B®, Cl-Sepharose-4B®, and AH-Sepharose-4B®.

5. The method of claims 1 or 2 wherein pepsin immobilization using the dicarboxylic acid disuccinimidyl ester of general formula (I) as the coupling agent is carried out in a buffer with pH comprised between

5.0 and 6.2.

6. The method of claim 5 wherein said buffer is phosphate buffer pH 6.0-6.2.

7. The method of claims 1 or 2 wherein pepsin immobilization using the dicarboxylic acid disuccinimidyl ester of general formula (I) as the coupling agent is carried out at a temperature comprised between 1 and 10 °C.

8. The method of claim 7 wherein said temperature is comprised between 2 and 6 °C.

9. The method of claims 1 or 2 wherein R is an alkylene radical containing from 1 to 10 carbon atoms.

10. The method of claim 9 wherein R is a straight alkylene radical containing from 2 to 6 carbon atoms.

11. A method for the preparation of lyophilized immunoglobulins for intravenous administration using pepsin immobilized on a hydrophilic gel functionalized with amino groups which comprises
   a) immobilizing pepsin on a hydrophilic gel suitably functionalised with amino groups, using a dicarboxylic acid disuccinimidyl ester of formula (I), wherein R is as defined above, as the coupling agent;
   b) incubating the obtained insolubilized pepsin with immunoglobulins;
   c) recovering the immunoglobulins and lyophilizing them.

12. The method of claim 11 wherein incubation with pepsin is carried out at a temperature comprised between 34 and 40 °C and pH comprised between 3.8 and 4.3.

13. The method of claim 12 wherein the pH is about 4.

14. The method of claim 12 wherein the incubation with pepsin lasts for 16-22 hours.

15. The method of claim 11 wherein incubation with pepsin is carried out with a protein concentration comprised between 1 and 5 % (w/v) and an enzymatic activity comprised between 600 and 3000 U/g of protein.

16. The method of claim 15 wherein protein concentration is comprised between 2 and 3 % (w/v).

**Revendications**

1. Procédé de préparation d'immunoglobulines pour administration intraveineuse, par traitement de celles-ci avec une pepsine immobilisée sur un gel hydrophile fonctionnalisé avec des groupes amino, caractérisé par le fait que l'on utilise comme agent de couplage un ester disuccinimidyle d'acide dicarboxylique de formule générale (I)

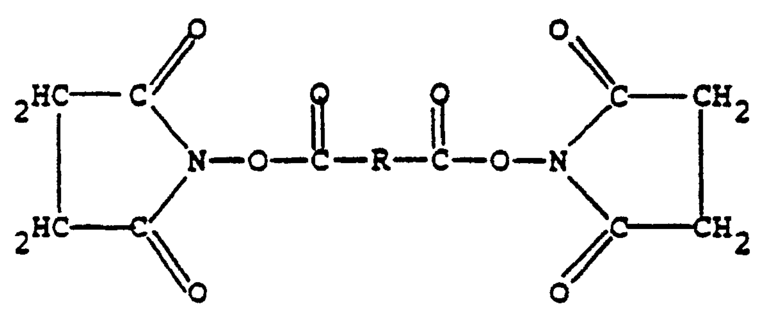

(I)

dans laquelle R est un radical alkylène linéaire ou ramifié contenant de 1 à 20 atomes de carbone, un groupe

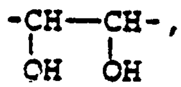 ou un groupe 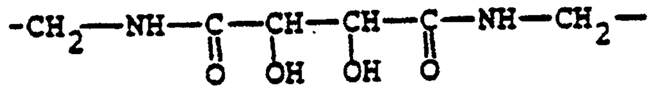

2. Procédé de préparation de pepsine immobilisée sur un gel hydrophile contenant des groupes amino, caractérisé par le fait que l'on utilise comme agent de couplage un ester disuccinimidyle d'acide dicarboxylique de formule générale (I)

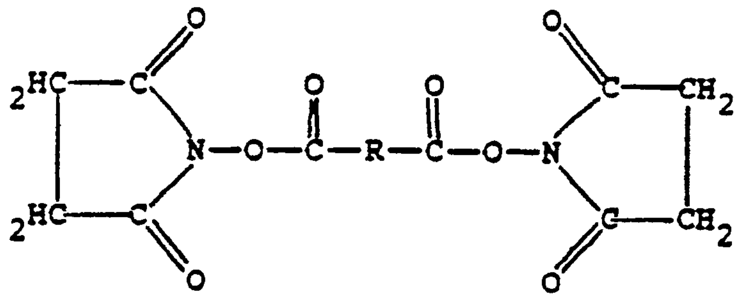 (I)

dans laquelle R est un radical alkylène linéaire ou ramifié contenant de 1 à 20 atomes de carbone, un groupe

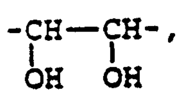 ou un groupe 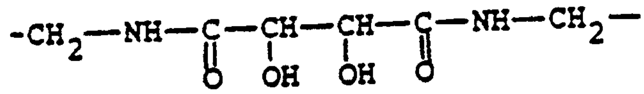

3. Procédé selon les revendications 1 ou 2, dans lequel le gel hydrophile est choisi parmi le gel d'agarose, le gel de dextrane, le gel de cellulose, le gel de polyacrylamide, le gel de silice poreuse, leurs dérivés aminés et un gel de polymère synthétique.

4. Procédé selon la revendication 3, dans lequel le gel hydrophile est choisi parmi le Sepharose-4B®, le Cl-Sepharose-4B® et le AH-Sepharose-4B®.

5. Procédé selon les revendications 1 ou 2, dans lequel l'immobilisation de la pepsine utilisant l'ester disuccinimidyle d'acide dicarboxylique de formule générale (I) en tant qu'agent de couplage est réalisée dans un milieu tampon de pH compris entre 5,0 et 6,2.

6. Procédé selon la revendication 5, dans lequel ledit milieu tampon est un tampon au phosphate de pH compris entre 6,0 et 6,2.

7. Procédé selon les revendications 1 ou 2, dans lequel l'immobilisation de la pepsine utilisant l'ester disuccinimidyle d'acide dicarboxylique de formule générale (I) en tant qu'agent de couplage est réalisée à une température comprise entre 1 °C et 10 °C.

8. Procédé selon la revendication 7, dans lequel la température est comprise entre 2 °C et 6 °C.

9. Procédé selon les revendications 1 ou 2, dans lequel R est un radical alkylène contenant de 1 à 10 atomes de carbone.

10. Procédé selon la revendication 9, dans lequel R est un radical alkylène linéaire contenant de 2 à 6 atomes de carbone.

11. Procédé de préparation d'immunoglobulines lyophilisées, pour administration intraveineuse, utilisant

une pepsine immobilisée sur un gel hydrophile fonctionnalisé avec des groupes amino, qui comprend :

a) l'immobilisation de la pepsine sur un gel hydrophile convenablement fonctionnalisé avec des groupes amino, utilisant un ester disuccinimidyle d'acide dicarboxylique de formule (I), dans lequel R est tel que défini plus haut, en tant qu'agent de couplage ;

b) l'incubation de la pepsine insolubilisée obtenue avec les immunoglobulines ;

c) la récupération des immunoglobulines et leur lyophilisation.

**12.** Procédé selon la revendication 11, dans lequel l'incubation avec la pepsine est effectuée à une température comprise entre 34° C et 40° C et avec un pH compris entre 3,8 et 4,3.

**13.** Procédé selon la revendication 12, dans lequel le pH vaut environ 4.

**14.** Procédé selon la revendication 12, dans lequel l'incubation avec la pepsine dure de 16 à 22 heures.

**15.** Procédé selon la revendication 11, dans lequel l'incubation avec la pepsine est effectuée avec une concentration en protéine comprise entre 1% et 5% (poids/volume) et une activité enzymatique comprise entre 600 et 3000 U/g de protéine.

**16.** Procédé selon la revendication 15, dans lequel la concentration en protéine est comprise entre 2% et 3% (poids/volume).

**Patentansprüche**

**1.** Verfahren zur Herstellung von Immunoglobulinen zur intravenösen Verabreichung durch Behandlung derselben mit Pepsin, das auf einem mit Aminogruppen funktionalisierten hydrophilen Gel immobilisiert ist, dadurch gekennzeichnet, daß ein Dicarbonsäuredisuccinimidylester der allgemeinen Formel (I)

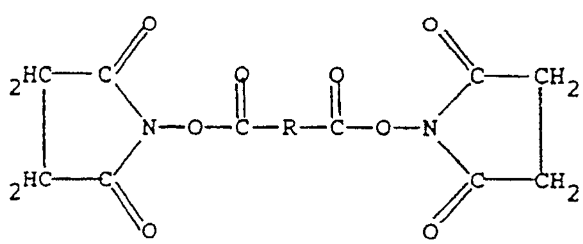

(I),

worin R einen gerad- oder verzweigtkettigen Alkylenrest, der 1 bis 20 Kohlenstoffatome enthält, eine

$-\underset{OH}{\underset{|}{CH}}-\underset{OH}{\underset{|}{CH}}-$ oder eine $-CH_2-NH-\underset{O}{\underset{\|}{C}}-\underset{OH}{\underset{|}{CH}}-\underset{OH}{\underset{|}{CH}}-\underset{O}{\underset{\|}{C}}-NH-CH_2-$Gruppe bedeutet,

als Haftvermittler verwendet wird.

**2.** Verfahren zur Herstellung von Pepsin, das auf einem Aminogruppen enthaltenden hydrophilen Gel immobilisiert ist, dadurch gekennzeichnet, daß ein Dicarbonsäuredisuccinimidylester der allgemeinen Formel (I)

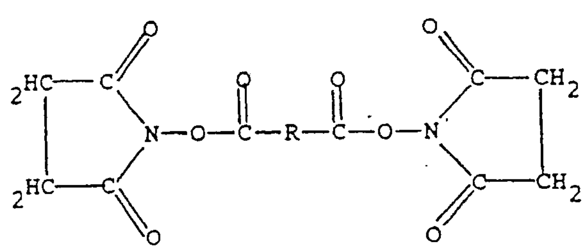

(I),

worin R einen gerad- oder verzweigtkettigen Alkylenrest, der 1 bis 20 Kohlenstoffatome enthält, eine

$$-\underset{OH}{\underset{|}{CH}}-\underset{OH}{\underset{|}{CH}}-\text{ oder eine }-CH_2-NH-\underset{O}{\underset{||}{C}}-\underset{OH}{\underset{|}{CH}}-\underset{OH}{\underset{|}{CH}}-\underset{O}{\underset{||}{C}}-NH-CH_2-\text{Gruppe bedeutet,}$$

als Haftvermittler verwendet wird.

3. Verfahren nach Anspruch 1 oder 2, wobei das hydrophile Gel aus Agarosegel, Dextrangel, Cellulosegel, Polyacrylamidgel, porösem Silikagel, deren aminierten Derivaten und synthetischem Polymergel ausgewählt wird.

4. Verfahren nach Anspruch 3, wobei das hydrophile Gel aus Sepharose-4B®, Cl-Sepharose-4B® und AH-Sepharose-4B® ausgewählt wird.

5. Verfahren nach Anspruch 1 oder 2, worin die Pepsinimmobilisierung unter Verwendung des Dicarbonsäuredisuccinimidylesters der allgemeinen Formel (I) als Haftvermittler in einem Puffer mit einem pH zwischen 5,0 und 6,2 durchgeführt wird.

6. Verfahren nach Anspruch 5, wobei der Puffer ein Phosphatpuffer mit pH 6,0 bis 6,2 ist.

7. Verfahren nach Anspruch 1 oder 2, worin die Pepsinimmobilisierung unter Verwendung des Dicarbonsäuredisuccinimidylesters der allgemeinen Formel (I) als Haftvermittler bei einer Temperatur zwischen 1 und 10° C durchgeführt wird.

8. Verfahren nach Anspruch 7, worin die Temperatur zwischen 2 und 6° C beträgt.

9. Verfahren nach Anspruch 1 oder 2, worin R einen Alkylenrest darstellt, der 1 bis 10 Kohlenstoffatome enthält.

10. Verfahren nach Anspruch 1 oder 2, worin R einen geradkettigen Alkylenrest darstellt, der 2 bis 6 Kohlenstoffatome enthält.

11. Verfahren zur Herstellung von lyophilisierten Immunoglobulinen zur intravenösen Verabreichung unter Verwendung von Pepsin, das auf einem mit Aminogruppen funktionalisierten hydrophilen Gel immobilisiert ist, umfassend:
   a) Immobilisieren des Pepsins auf einem mit Aminogruppen geeignet funktionalisierten hydrophilen Gel unter Verwendung eines Dicarbonsäuredisuccinimidylesters der allgemeinen Formel (I), worin R wie oben definiert ist, als Haftvermittler;
   b) Inkubieren des erhaltenen unlöslich gemachten Pepsins mit Immunoglobulinen;
   c) Gewinnen der Immunoglobuline und Lyophilisieren derselben.

12. Verfahren nach Anspruch 11, worin die Inkubation mit Pepsin bei einer Temperatur zwischen 34 und 40° C und einem pH zwischen 3,8 und 4,3 durchgeführt wird.

13. Verfahren nach Anspruch 12, worin der pH etwa 4 beträgt.

14. Verfahren nach Anspruch 12, worin die Inkubation mit Pepsin 16 bis 22 Stunden dauert.

15. Verfahren nach Anspruch 11, worin die Inkubation mit Pepsin mit einer Proteinkonzentration zwischen 1 und 5 % (G/V) und einer enzymatischen Aktivität zwischen 600 und 3000 E/g Protein durchgeführt wird.

16. Verfahren nach Anspruch 15, worin die Proteinkonzentration zwischen 2 und 3 % (M/V) beträgt.